(19) Europäisches Patentamt
European Patent Office
Office européen des brevets

(11) Publication number: **0 154 780**
**B1**

(12) # EUROPEAN PATENT SPECIFICATION

(45) Date of publication of patent specification: **11.04.90**

(21) Application number: **85100767.4**

(22) Date of filing: **25.01.85**

(51) Int. Cl.⁵: **C 07 D 215/56,** C 07 D 401/04, C 07 D 405/04, C 07 D 409/04, C 07 D 417/04, C 07 D 491/056, C 07 D 401/14, C 07 D 405/14, C 07 D 409/14, C 07 D 417/14, A 61 K 31/47

(54) **Quinoline antibacterial compounds.**

(30) Priority: **26.01.84 US 574119**
**26.01.84 US 574225**
**26.01.84 US 574224**

(43) Date of publication of application:
**18.09.85 Bulletin 85/38**

(45) Publication of the grant of the patent:
**11.04.90 Bulletin 90/15**

(84) Designated Contracting States:
**BE CH DE FR GB IT LI NL SE**

(56) References cited:
**EP-A-0 126 355** **GB-A-2 094 305**
**EP-A-0 131 839** **US-A-4 017 622**
**DE-A-2 021 100** **US-A-4 284 629**
**GB-A-1 147 336** **US-A-4 292 317**

**Progress in Drug Research, vol. 21, 1977, Basel R.Albrecht "Antibacterial Agents" pages 12-14**

**The file contains technical information submitted after the application was filed and not included in this specification**

(73) Proprietor: **ABBOTT LABORATORIES**
**14th Street and Sheridan Road North St**
**North Chicago Illinois 60064 (US)**

(72) Inventor: **Tim-Wo Chu, Daniel**
**204 Ashland Court**
**Vernon Hills Illinois (US)**

(74) Representative: **Modiano, Guido et al**
**MODIANO, JOSIF, PISANTY & STAUB Modiano & Associati Via Meravigli, 16**
**I-20123 Milano (IT)**

**EP 0 154 780 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to new quinoline derivatives having antibacterial properties, compositions containing the new quinoline derivatives and methods of treating mammalian patients with the new quinoline derivatives.

It is known that certain quinoline compounds exhibit antibacterial properties, notably certain 7-piperazinyl-4-oxo-1,4-dihydroquinoline-3-carboxylic acids. In U.S. Patent No. 4,017,622, there are disclosed certain 7-piperazinyl-4-oxo-1,4-dihydroquinoline-3-carboxylic acids derivatives which are substituted in the 1 position with an alkyl, benzyl or acetyl substituent. U.S. Patent No. 4,292,317 discloses derivatives of 7-piperazinyl-4-oxo-1,4-dihydroquinoline-3-carboxylic acid wherein the 1 position is substituted by an alkyl group or a vinyl group. In U.S. Patent No. 4,284,629, there are disclosed various 4-oxo-1,4-dihydroquinoline-3-carboxylic acids in which the 1 position is substituted with a cycloalkyl group.

Other quinoline derivatives are disclosed in EP—A—131 839, US—A—4,017,622 or in Progress in Drug Research 21/12977) 12—14, which however are not 6,8-difluoro 1-phenyl substituted quinolines. GB—A—2 094 305 discloses quinoline derivatives having an ethyl substituent in the 1-position while GB—A—1 147 336 discloses quinoline derivatives which are not 6,8-difluoro substituted.

This invention relates to novel antibacterial agents having the formula:

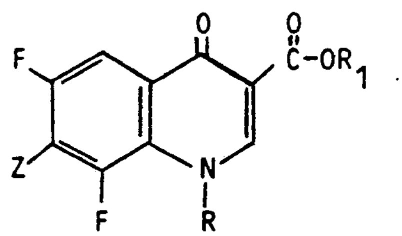

wherein $R_1$ is hydrogen or a carboxy protecting group; R is phenyl having the formula:

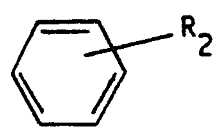

wherein $R_2$ is methylenedioxy or one or two substituents independently selected from hydrogen, halogen, nitro, carboxyl cyano, $C_1$ to $C_6$ alkyl, hydroxy-substituted $C_1$ to $C_6$ alkyl, halo substituted $C_1$ to $C_6$ alkyl, a substituent having the formula

$$—Y—R_3$$

wherein —Y— is —O— or —S— and $R_3$ is hydrogen or $C_1$ to $C_6$ alkyl, and a substituent of the formula:

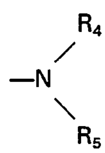

wherein $R_4$ and $R_5$ are independently hydrogen or $C_1$ to $C_6$ alkyl; and, Z is selected from 1) an aliphatic heterocyclic ring having the structure:

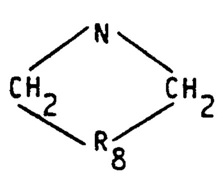

wherein $R_8$ is $—CH_2—CH_2—$ or $—CH_2—R_9—CH_2—$ wherein $R_9$ is selected from the group consisting of —S, —O—, —NH—, $—CH_2—$ and $—NH—CH_2—$; 2) substituted derivatives of said aliphatic heterocyclic ring having one or two substituents independently selected from $C_1$ to $C_6$ alkyl, $C_1$ to $C_6$ hydroxyalkyl, hydroxy, alkanoyl having 1 to 6 carbon atoms, alkanoylamino having 1 to 6 carbon atoms and a substituent of the formula:

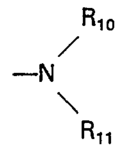

wherein $R_{10}$ and $R_{11}$ are each independently selected from hydrogen and $C_1$ to $C_6$ alkyl; and 3) a substituent of the formula:

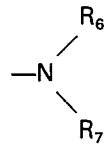

wherein $R_6$ is hydrogen, $C_1$ to $C_{10}$ alkyl or hydroxy-substituted $C_1$ to $C_{10}$ alkyl, and $R_7$ is selected from $C_1$ to $C_{10}$ alkyl, hydroxy-substituted $C_1$ to $C_{10}$ alkyl, $—NH_2$, a mono-($C_1$ to $C_6$) alkylamino and a di-($C_1$ to $C_6$) alkylamino; and pharmaceutically acceptable salts thereof.

As used herein, the term "halogen" refers to chloro, bromo, fluoro and iodo groups, while the term "$C_1$ to $C_6$ alkyl" refers to lower alkyl groups including methyl, ethyl, propyl, isopropyl and butyl.

As indicated above, $R_2$ can be $C_1$ to $C_6$ straight or branched alkyl as well as hydroxy and halo-substituted derivatives thereof. Such groups include a chloromethyl group, a chloroethyl group, a chloropropyl group, a hydroxyethyl group, and a trifluoromethyl group.

$R_2$ can also be a group of the formula $—Y—R_3$. Representative groups of this type include a hydroxy group, a mercapto group, a lower alkoxy group, such as methoxy, ethoxy, propoxy, as well as the thio analogs thereof, namely a methylmercapto group, and an ethylmercapto group.

As used herein, the term "carboxy-protecting group" refers to and includes the residue of a carboxylic acid ester group. Such carboxy-protecting groups are well known to those skilled in the art, having been extensively used in the protection of carboxyl groups in the penicillin and cephalosporin fields, as described in U.S. Patent Nos. 3,840,556 and 3,719,667. In general, such carboxy-protecting groups can be relatively easily cleaved to yield the corresponding free carboxy group. Representative protecting groups include $C_1—C_8$ alkyl (e.g., methyl, ethyl, tertiary butyl), benzyl and substituted derivatives thereof such as alkoxy and nitrobenzyl groups; also suitable are acyl groups such as pivaloyloxymethyl groups.

Illustrative of aliphatic heterocyclic groups represented by Z are piperazinyl groups, 4-acylpiperazinyl groups, piperidinyl groups, pyrrolidinyl groups, morpholino groups, thiomorpholino groups and homopiperazinyl groups (i.e., hexahydro-1-H-1,4-diazepinyl).

Also included within the scope of the present invention are pharmaceutically acceptable salts of the foregoing compounds. As used herein, the term "pharmaceutically acceptable salts" refers to non-toxic acid addition salts and alkaline earth metal salts of the compounds of formula I. The salts can be prepared *in situ* during the final isolation and purification of the compounds of formula I, or separately by reacting the free base or acid functions with a suitable organic acid or base. Representative acid addition salts include the hydrochloride, hydrobromide, sulphate, bisulphate, acetate, oxalate, valerate, oleate, palmitate, stearate, laurate, borate, benzoate, lactate, phosphate, tosylate, mesylate, citrate, maleate, fumarate, succinate, tartrate, glucoheptonate, lactobionate, lauryl sulfate salts and the like. Representative alkali or alkaline earth metal salts include the sodium, calcium, potassium and magnesium salts. It has been found that the compounds of the present invention possess antibacterial activity against a wide spectrum of gram positive and gram negative bacteria, as well as enterobacteria. The compounds of the invention are therefor useful in the antibiotic treatment of susceptible bacterial infections in both humans and animals. In addition, the compounds, by reason of their *in vitro* activity, may be used in scrub solutions for surface inhibition of bacterial growth.

Susceptible organisms generally include those gram positive and gram negative, aerobic and anaerobic organisms whose growth can be inhibited by the compounds of the invention such as *Staphylococcus, Lactobacillus, Streptococcus, Sarcina, Escherichia, Enterobacter, Klebsiella, Pseudomonas, Acinetobacter, Proteus, Citrobacter, Nisseria, Baccillus, Bacteroides, Peptococcus, Clostridium, Salmonella, Shiqella, Serratia, Haemophilus, Brucella,* and other organisms. In addition to exhibiting highly effective antibacterial activity, the compounds of the invention exhibit increased and improved solubility characteristics as compared with prior quinoline-3-carboxylic acid compounds in the art.

The compounds of Formula I may also be formulated into compositions together with pharmaceutically acceptable carriers for parenteral injection, for oral administration in solid or liquid form, for rectal administration, and the like.

Compositions according to the invention for parenteral injection may comprise pharmaceutically acceptable sterile aqueous or nonaqueous solutions, suspensions or emulsions. Examples of suitable nonaqueous carriers, diluents, solvents or vehicles include propylene glycol, polyethylene glycol, vegetable oils, such as olive oil, and injectable organic esters such as ethyl oleate. Such compositions may also contain adjuvants such as preserving, wetting, emulsifying, and dispersing agents. They may be

sterilized, for example, by filtration through a bacteria-retaining filter, or by incorporating sterilizing agents into the compositions. They can also be manufactured in the form of sterile solid compositions which can be dissolved in sterile water, or some other sterile injectable medium immediately before use.

Solid dosage forms for oral administration include capsules, tablets, pills, powders and granules. In such solid dosage forms, the active compound is admixed with at least one inert diluent such as sucrose, lactose or starch. Such dosage forms can also comprise, as is normal practice, additional substances other than diluents, e.g., lubricating agents such as magnesium stearate. In the case of capsules, tablets and pills, the dosage forms may also comprise buffering agents. Tablets and pills can additionally be prepared with enteric coatings.

Liquid dosage forms for oral administration include pharmaceutically acceptable emulsions, solutions, suspensions, syrups and elixers containing inert diluents commonly used in the art, such as water. Besides such inert diluents, compositions can also include adjuvants, such as wetting agents, emulsifying and suspending agents, and sweetening, flavoring and perfuming agents.

Compositions for rectal administration are preferably suppositories which may contain, in addition to the active substance, excipients such as cocoa butter or a suppository wax.

Actual dosage levels of active ingredient in the compositions of the invention may be varied so as to obtain an amount of active ingredient effective to achieve antibacterial activity in accordance with the desired method of administration. The selected dosage level therefore depends upon the nature of the active compound administered, the route of administration, the desired duration of treatment and other factors. Generally, daily dosage levels of the compounds of Formula I of about 0.1 to about 750, more preferably about 0.25 to about 500 and most preferably about 0.5 to about 300 mg. of active ingredient per kg. of body weight are effective when administered orally to a mammalian patient suffering from an infection caused by a susceptible organism. If desired, the daily dose may be divided into multiple doses for administration, e.g., two to four times per day.

The foregoing may be better understood from the following examples, which are presented for purposes of illustration and are not intended to limit the scope of the inventive concepts.

## Example 1

1-phenyl-6,8-difluoro-1,4-dihydro-4-oxo-7-(1-piperazinyl)-quinoline-3-carboxylic acid

(a) To a cold solution of 30 g., 2,3,4,5-tetrafluoroacetophenone in 400 ml. diethylcarbonate is slowly added of 12.5 g. 60% sodium hydride-in-oil suspension. The mixture is heated at 80°C for 1—1/2 hours, then poured into 700 ml. ice cold water solution containing 25 ml. acetic acid. The mixture is extracted with three 400 ml. portions of ether. The organic phase is dried over $MgSO_4$, evaporated and the obtained liquid is distilled to give ethyl 2,3,4,5-tetrafluorobenzoyl acetate.

(b) A solution of 20 g. of (a) in 18.5 ml. of triethylorthoformate and 45 ml. of acetic anhydride is heated at 135°C for 1—1/2 hours with the removal of the ethyl acetate formed during the reaction. The solution is evaporated under reduced pressure to a mobile oil. The oil is then dissolved in 200 ml. of methylene chloride and 9.9 ml. of aniline is added into the solution. After 1 hour, the solution is evaporated to dryness and crystallized from 200 ml. of hexane and 5 ml. of ether yielding ethyl 3-anilino-2-(2,3,4,5-tetrafluorobenzoyl)acrylate.

(c) To a cold solution of 15 g. of the preceding acrylate in 150 ml. tetrahydrofuran (THF) is slowly added 1.63 g. of a 60% sodium hydride-in-oil suspension. The mixture is refluxed for 6 hours and is cooled and diluted with water to a volume of 1.5 liters. The mixture is then filtered and the solid is washed with a 1:1 hexane/ether solution to obtain ethyl 1-phenyl-6,7,8-trifluoro-1,4-dihydro-4-oxo-quinoline-3-carboxylate.

(d) To a suspension of 7 g. of the product (c) in 30 ml. THF is added a sodium hydroxide solution (0.88 g. in 20 ml. $H_2O$). The mixture is heated at 80°C for 1 hour resulting in a clear solution which is evaporated under reduced pressure to dryness. The solid is dissolved in 200 ml. $H_2O$, and 2.5 ml. acetic acid is added. The resulting precipitate is filtered and washed with cold water, crystallized from dimethylformamide (DMF) to produce 1-phenyl-6,7,8-trifluoro-1,4-dihydro-4-oxo-quinoline-3-carboxylic acid.

(e) To a solution of 2.5 g. of 1-phenyl-6,7,8-trifluoro-1,4-dihydro-4-oxo-quinoline-3-carboxylic acid in 15 ml. of 1-methyl-2-pyrrolidinone at 115°C is added 3 ml. piperazine. After stirring at 100°C for 20 hours, the solvent is removed by reduced pressure to dryness. Ethanol is added to the residue and the resulting mixture is filtered and washed with ether and then washed with very small amounts of cold water to give a solid. This is suspended in 30 ml. $H_2O$ and 7.8 ml. 1N HCl is added to and warmed to dissolve. Removal of the solvent under reduced pressure gives hydrochloride salt of 1-phenyl-6,8-difluoro-1,4-dihydro-4-oxo-7-(1-piperazinyl)quinoline-3-carboxylic acid.

To the hydrochloride salt is added one molar equivalent of an aqueous solution of sodium hydroxide, and the resulting precipitate is filtered to obtain 1-phenyl-6,8-difluoro-1,4-dihydro-4-oxo-7-(1-piperazinyl)-quinoline-3-carboxylic acid.

(f) Alternately, the title compound is prepared as follows: To a suspension of 5 g. of the product of 1(d) in 40 ml. 1-methyl-2-pyrrolidinone at 120° under nitrogen atmosphere is added 9.5 ml. of N-carboethoxy-piperazine. After 20 hours, the solvent is removed under reduced pressure, and the residue is suspended in 150 ml. ethanol and refluxed for ½ hour. The reaction mixture is then cooled and filtered. The resulting solid is washed with cold ethanol and water to obtain 1-phenyl-6,8-difluoro-7-(4-carboethoxy-1-piperazinyl)-1,4-dihydro-4-oxo-quinoline-3-carboxylic acid.

To a suspension of 5 g. of the preceding compound in 25 ml. of ethanol at 80°C is added 50 ml. of 10% NaOH solution. The solution is heated at 80°C for 6 hours. The solvent is removed and the solid is dissolved in 100 ml. water. The pH of the solution is adjusted to pH 7 by the addition of 10% acetic acid. The precipitate is filtered and washed with cold water yielding 1-phenyl-6,8-difluoro-1,4-dihydro-4-oxo-7-(piperazinyl)-quinoline-3-carboxylic acid.

(g) Alternatively, to the process of 1(a), the ethyl-2,3,4,5-tetrafluorobenzoyl acetate is prepared as follows: To a solution of 39 g. of 2,3,4,5-tetrafluorobenzoic acid in 200 ml. of methylene chloride are added 40 ml. of thionyl chloride and 2 drops of dimethylformamide. After refluxing for 3 hours, the reaction mixture is evaporated to dryness to give the corresponding acid chloride.

To a dry ice-cooled solution of 35 g. of malonic acid monoethyl ester in 350 ml. of tetrahydrofuran is added 379 ml. of 1.4 M. n-butyl lithium in THF, and the flask is warmed to −5°C. for 5 minutes and then is cooled back to −70°C. To the resulting solution there is added the above-prepared acid chloride in 100 ml. of THF. The cooling bath is removed, and the solution allowed to warm to room temperature after an hour. The solution is then partitioned between 1 N HCl and ether. The ether portion is washed with $NaHCO_3$ and dried over $MgSO_4$, and then evaporated to obtain the ethyl 2,3,4,5-tetrafluorobenzoylacetate.

Example 2

1-phenyl-6,8-difluoro-1,4-dihydro-4-oxo-7-(1-4-methyl)piperazinyl)-quinoline-3-carboxylic acid

The procedure of Example 1 is repeated replacing piperazine in Example 1(e) with N-methylpiperazine to obtain 1-phenyl-6,8-difluoro-1,4-dihydro-4-oxo-7-(1-(4-methyl)piperazinyl)-quinoline-3-carboxylic acid and its hydrochloride salt.

Example 3

1-phenyl-6,8-difluoro-1,4-dihydro-4-oxo-7-(1-pyrrolidinyl)-quinoline-3-carboxylic acid

In the described fashion as Example 1, replacing piperazine in Example 1(e) with pyrrolidine, one can obtain 1-phenyl-6,8-difluoro-1,4-dihydro-4-oxo-7-(1-pyrrolidinyl)quinoline-3-carboxylic acid in good yield.

Example 4

1-phenyl-6,8-difluoro-1,4-dihydro-4-oxo-7-(3-hydroxy-1-pyrrolidinyl)-quinoline-3-carboxylic acid

The procedure of Example 1 can be repeated replacing piperazine in Example 1(e) with 3-hydroxypyrrolidine to obtain 1-phenyl-6,8-difluoro-1,4-dihydro-4-oxo-7-(3-hydroxy-1-pyrrolidinyl)-quinoline-3-carboxylic acid.

Example 5

1-phenyl-6,8-difluoro-1,4-dihydro-4-oxo-7-(3-amino-1-pyrrolidinyl)-quinoline-3-carboxylic acid

(a) In the described fashion as Example 1 replacing piperazine in Example 1(e) with 3-acetamidopyrrolidine, one can obtain 1-phenyl-6,8-difluoro-1,4-dihydro-4-oxo-7-(3-acetamido-1-pyrrolidinyl)-quinoline-3-carboxylic acid in good yield.

(b) The product of above reaction can then be hydrolyzed with hydrochloric acid at 80°C to give 1-phenyl-6,8-difluoro-1,4-dihydro-4-oxo-7-(3-amino-1-pyrrolidinyl)-quinoline-3-carboxylic acid hydrochloride salt.

Example 6

1-phenyl-6,8-difluoro-1,4-dihydro-4-oxo-7-(1-piperidinyl)-quinoline-3-carboxylic acid

The procedure of Example 1 is repeated replacing piperazine in Example 1(e) with piperidine to obtain 1-phenyl-6,8-difluoro-1,4-dihydro-4-oxo-7-(1-piperidinyl)-quinoline-3-carboxylic acid.

Example 7

1-phenyl-6,8-difluoro-1,4-dihydro-4-oxo-7-(4-morpholinyl)-quinoline-3-carboxylic acid

In the described fashion as Example 1, replacing piperazine in Example 1(e) with morpholine, one can obtain 1-phenyl-6,8-difluoro-1,4-dihydro-4-oxo-7-(4-morpholinyl)-quinoline-3-carboxylic acid in good yield.

Example 8

1-phenyl-6,8-difluoro-1,4-dihydro-4-oxo-7-(4-thiomorpholinyl)-quinoline-3-carboxylic acid

The procedure of Example 1 can be repeated replacing piperazine in Example 1(e) with thiomorpholine to obtain 1-phenyl-6,8-difluoro-1,4-dihydro-4-oxo-7-(4-thiomorpholinyl)-quinoline-3-carboxylic acid.

Example 9

1-phenyl-6,8-difluoro-1,4-dihydro-4-oxo-7-(3,5-dimethyl-1-piperazinyl)-quinoline-3-carboxylic acid

In the described fashion as Example 1, replacing piperazinyl in Example 1(e) with 2,6-dimethylpiperazine, one obtains 1-phenyl-6,8-difluoro-1,4-dihydro-4-oxo-7-(3,5-dimethyl-1-piperazinyl)-quinoline-3-carboxylic acid and its hydrochloride salt.

Example 10

1-phenyl-6,8-difluoro-1,4-dihydro-4-oxo-7-(1-homopiperazinyl)-quinoline-3-carboxylic acid

The procedure of Example 1 is repeated replacing piperazine in Example 1(e) with homopiperazine to obtain 1-phenyl-6,8-difluoro-4-dihydro-4-oxo-7-(1-homopiperazinyl)-quinoline-3-carboxylic acid and its hydrochloride salt.

Example 11

1-phenyl-6,8-difluoro-1,4-dihydro-4-oxo-7-(dimethylamino)-quinoline-3-carboxylic acid

In the described fashion as Example 1, replacing piperazine in Example 1(e) with dimethylamine, one can obtain 1-phenyl-6,8-difluoro-1,4-dihydro-4-oxo-7-(dimethylamino)-quinoline-3-carboxylic acid.

Example 12

1-phenyl-6,8-difluoro-1,4-dihydro-4-oxo-7-(N-2-hydroxyethylamino)-quinoline-3-carboxylic acid

The procedure of Example 1 can be repeated replacing piperazine in Example 1(e) with N-2-hydroxyethylamine to obtain 1-phenyl-6,8-difluoro-1,4-dihydro-4-oxo-7-(N-2-hydroxy-ethylamino)-quinoline-3-carboxylic acid.

Example 13

1-phenyl-6,8-difluoro-1,4-dihydro-4-oxo-7-hydrazyl-quinoline-3-carboxylic acid

In the described fashion as Example 1, replacing piperazine in Example 1(e) with hydrazine, one can obtain 1-phenyl-6,8-difluoro-1,4-dihydro-4-oxo-7-(hydrazyl)-quinoline-3-carboxylic acid and its hydrochloride salt.

Example 14

1-phenyl-6,8-difluoro-1,4-dihydro-4-oxo-7-(2,2-dimethylhydrazyl)-quinoline-3-carboxylic acid

The procedure of Example 1 can be repeated, replacing piperazine in Example 1(e) with 1,1-dimethylhydrazine to obtain 1-phenyl-6,8-difluoro-1,4-dihydro-4-oxo-7-(2,2-dimethyl-hydrazyl)-quinoline-3-carboxylic acid and its hydrochloride salt.

Example 15

1-p-fluorophenyl-6,8-difluoro-1,4-dihydro-4-oxo-7-(1-piperazinyl)-quinoline-3-carboxylic acid

(a) In the described fashion as Example 1(b), replacing aniline with p-fluoroaniline, one can obtain ethyl 3-(p-fluoroanilino)-2-(2,3,4,5-tetrafluorobenzoyl)acrylate.

(b) By following the Example 1(c) and 1(d), the preceding compound (a) can yield 7-fluoro-1-p-fluorophenyl-6,8-difluoro-1,4-dihydro-4-oxo-quinoline-3-carboxylic acid.

(c) In the described fashion as Example 1(e), the above product of (b) can give the desired 1-p-fluorophenyl-6,8-difluoro-1,4-dihydro-4-oxo-7-(1-piperazinyl)-quinoline-3-carboxylic acid and its hydrochloride salt.

Example 16

In the described fashion as Example 1(e), replacing the acid with the acid of the product of Example 15(b) and also replacing piperazine with an appropriate amine such as n-methylpiperazine, pyrrolidine, 3-hydroxypyrrolidine, 3-acetaminopyrrolidine, piperidine, morpholine, thiomorpholine, 2,6-dimethylpiperazine, homopiperazine, diethylamine and 2,2-dimethylhydrazine, one can obtain the following compounds:

(a) 1-p-fluorophenyl-6,8-difluoro-1,4-dihydro-4-oxo-7-(1-(4-methyl)piperazinyl)-quinoline-3-carboxylic acid.

(b) 1-p-fluorophenyl-6,8-difluoro-1,4-dihydro-4-oxo-7-(1-pyrrolidinyl)-quinoline-3-carboxylic acid.

(c) 1-p-fluorophenyl-6,8-difluoro-1,4-dihydro-4-oxo-7-(3-hydroxy-1-pyrrolidinyl)-quinoline-3-carboxylic acid.

(d) 1-p-fluorophenyl-6,8-difluoro-1,4-dihydro-4-oxo-7-(3-acetamino-1-pyrrolidinyl)-quinoline-3-carboxylic acid.

(e) 1-p-fluorophenyl-6,8-difluoro-1,4-dihydro-4-oxo-7-(1-piperidinyl)-quinoline-3-carboxylic acid.

(f) 1-p-fluorophenyl-6,8-difluoro-1,4-dihydro-4-oxo-7-(4-morpholinyl)-quinoline-3-carboxylic acid.

(g) 1-p-fluorophenyl-6,8-difluoro-1,4-dihydro-4-oxo-7-(4-thiomorpholinyl)-quinoline-3-carboxylic acid.

(h) 1-p-fluorophenyl-6,8-difluoro-1,4-dihydro-4-oxo-7-(3,5-dimethyl-1-piperazinyl)-quinoline-3-carboxylic acid.

(i) 1-p-fluorophenyl-6,8-difluoro-1,4-dihydro-4-oxo-7-(1-homopiperazinyl)-quinoline-3-carboxylic acid.

(j) 1-p-fluorophenyl-6,8-difluoro-1,4-dihydro-4-oxo-7-(diethylamino)-quinoline-3-carboxylic acid.

(k) 1-p-fluorophenyl-6,8-difluoro-1,4-dihydro-4-oxo-7-(2,2-dimethylhydrazyl)-quinoline-3-carboxylic acid.

Example 17

1-p-fluorophenyl-6,8-difluoro-1,4-dihydro-4-oxo-7-(3-amino-1-pyrrolidinyl)-quinoline-3-carboxylic acid

In the described fashion of Example 5(b), the compound of Example 16(d) can give 1-p-fluorophenyl-6,8-difluoro-1,4-dihydro-4-oxo-7-(3-amino-1-pyrrolidinyl)-quinoline-3-carboxylic acid.

Example 18

1-p-hydroxyphenyl-6,8-difluoro-1,4-dihydro-4-oxo-7-(1-piperazinyl)-quinoline-3-carboxylic acid

(a) In the described fashion as Example 1(b), replacing aniline with hydroxyaniline, one can obtain ethyl 3-(p-hydroxyanilino)-2-(2,3,4,5-tetrafluorobenzoyl)acrylate.

(b) By following the Example 1(c) and 1(d), the preceding compound can yield 7-fluoro-1-p-hydroxyphenyl-6,8-difluoro-1,4-dihydro-4-oxo-quinoline-3-carboxylic acid.

(c) In the described fashion as Example 1(e), the above acid of (b) can give the desired 1-p-hydroxyphenyl-6,8-difluoro-1,4-dihydro-4-oxo-7-(1-piperazinyl)quinoline-3-carboxylic acid and its hydrochloride salt.

Example 19

In the described fashion as Example 1(e), replacing the acid with the acid of the product of Example 18(b) and also replacing piperazine with an appropriate amine such as N-methylpiperazine, pyrrolidine, 3-hydroxy pyrrolidine, 3-acetamidopyrrolidine, piperidine, morpholine, thiomorpholine, 2,6-dimethylpiperazine, homopiperazine, dimethylamine and 2,2-dimethylhydrazine, one can obtain the following compounds:

(a) 1-p-hydroxyphenyl-6,8-difluoro-1,4-dihydro-4-oxo-7-(1-(4-methyl)piperazinyl)-quinoline-3-carboxylic acid and its hydrochloride salt.

(b) 1-hydroxyphenyl-6,8-difluoro-1,4-dihydro-4-oxo-7-(1-pyrrolidinyl)-quinoline-3-carboxylic acid.

(c) 1-p-hydroxyphenyl-6,8-difluoro-1,4-dihydro-4-oxo-7-(3-hydroxy-1-pyrrolidinyl)-quinoline-3-carboxylic acid.

(d) 1-p-hydroxyphenyl-6,8-difluoro-1,4-dihydro-4-oxo-7-(3-acetamino-1-pyrrolidinyl)-quinoline-3-carboxylic acid.

(e) 1-p-hydroxyphenyl-6,8-difluoro-1,4-dihydro-4-oxo-7-(1-piperidinyl)-quinoline-3-carboxylic acid.

(f) 1-p-hydroxyphenyl-6,8-difluoro-1,4-dihydro-4-oxo-7-(4-morpholinyl)-quinoline-3-carboxylic acid.

(g) 1-p-hydroxyphenyl-6,8-difluoro-1,4-dihydro-4-oxo-7-(4-thiomorpholinyl)-quinoline-3-carboxylic acid.

(h) 1-p-hydroxyphenyl-6,8-difluoro-1,4-dihydro-4-oxo-7-(3,5-dimethyl-1-piperazinyl)-quinoline-3-carboxylic acid.

(i) 1-p-hydroxyphenyl-6,8-difluoro-1,4-dihydro-4-oxo-7-(1-homopiperazinyl)-quinoline-3-carboxylic acid.

(j) 1-p-hydroxyphenyl-6,8-difluoro-1,4-dihydro-4-oxo-7-(diethylamino)-quinoline-3-carboxylic acid.

(k) 1-p-hydroxyphenyl-6,8-difluoro-1,4-dihydro-4-oxo-7-(2,2-dimethyl-hydrazyl)-quinoline-3-carboxylic acid.

Example 20

1-p-hydroxyphenyl-6,8-difluoro-1,4-dihydro-4-oxo-7-(3-amino-1-pyrrolidinyl)-quinoline-3-carboxylic acid

In the described fashion of Example 5(b), the compound of Example 18(d) can give 1-p-hydroxyphenyl-6,8-difluoro-1,4-dihydro-4-oxo-7-(3-amino-1-pyrrolidinyl)-quinoline-3-carboxylic acid.

Example 21

In the described fashion as Example 1(a-d), replacing aniline with an appropriate amine ($R—NH_2$), one can obtain the additional 1-substituted 6,7,8-trifluoro-1,4-dihydro-4-oxo-quinoline-3-carboxylic acid as listed in Table I.

Table I

| | Aniline replacement | R-Substituent |
|---|---|---|
| a) | o-fluoroaniline | o-fluorophenyl |
| b) | p-chloroaniline | p-chlorophenyl |
| c) | o-chloroaniline | p-chlorophenyl |
| d) | p-methoxyaniline | p-methoxyphenyl |
| e) | p-methoxyaniline | p-methoxyphenyl |
| f) | 2,4-difluoroaniline | 2,4-difluorophenyl |
| g) | 2-hydroxyaniline | 2-hydroxyphenyl |
| h) | 2-hydroxy-4-fluoroaniline | 2-hydroxy-4-fluorophenyl |
| i) | 2,4-dihydroxyaniline | 2,4-dihydroxyphenyl |
| j) | p-cyanoaniline | p-cyanophenyl |
| k) | 2,4-diaminobenzene | p-aminophenyl |
| l) | p-dimethylaminoaniline | p-dimethylaminophenyl |
| m) | p-methylmercaptoaniline | p-methylmercaptophenyl |
| n) | p-aminothiophenol | p-mercaptophenyl |
| u) | 3-chloro-4-hydroxyaniline | 3-chloro-4-hydroxyphenyl |
| v) | 3,4-methylenedioxyaniline | 3,4-methylenedioxyphenyl |

Example 22

In the described fashion as Example 1(e), replacing the acid with the acid of the compounds listed in Table 1 of Example 21 and also replacing piperazine with an appropriate amine such as N-methylpiperazine, pyrrolidine, 3-hydroxypyrrolidine, 3-acetamidopyrrolidine, 3-dimethylaminopyrrolidine, piperidine, morpholine, thiomorpholine, 2,6-dimethylpiperazine, homopiperazine, dimethylamine and 2,2-dimethylhydrazine, one can obtain the following additional compounds as summarized in Table II.

Table II

| | Piperazine replacement ZH | R-Substituent of Product of Ex. 21 | Compound I obtained ($R_1$=H; W and X=F) R | Z |
|---|---|---|---|---|
| 1. | piperazine | o-fluorophenyl | o-fluorophenyl | piperazinyl |
| 2. | piperazine | p-methoxyphenyl | p-methoxyphenyl | piperazinyl |
| 3. | piperazine | p-mercaptophenyl | p-mercaptophenyl | piperazinyl |
| 4. | N-methylpiperazine | 2,4-difluorophenyl | 2,4-difluorophenyl | N-methylpiperazinyl |
| 5. | N-methylpiperazine | o-fluorophenyl | o-fluorophenyl | N-methylpiperazinyl |
| 6. | N-methylpiperazine | o-hydroxyphenyl | o-hydroxyphenyl | N-methylpiperazinyl |
| 7. | pyrrolidine | o-fluorophenyl | p-fluorophenyl | pyrrolidinyl |
| 8. | pyrrolidine | p-dimethylamino-phenyl | p-dimethylamino-phenyl | pyrrolidinyl |
| 9. | 3-hydroxy-pyrrolidine | o-fluorophenyl | o-fluoro-phenyl | 3-hydroxypyrrolidinyl |
| 10. | 3-hydroxy-pyrrolidine | p-chlorophenyl | p-chlorophenyl | 3-hydroxypyrrolidinyl |
| 11. | 3-acetamido-pyrrolidine | o-fluorophenyl | o-fluorophenyl | 3-acetamido-pyrrolidinyl |
| 12. | 3-acetamido-pyrrolidine | p-methylphenyl | p-methylphenyl | 3-acetamido-pyrrolidinyl |
| 13. | 3-dimethyl-pyrrolidinyl | p-fluorophenyl | p-fluorophenyl | 3-dimethylamino-pyrrolidinyl |
| 14. | piperidine | o-fluorophenyl | o-fluorophenyl | piperidinyl |

Table II (continued)

| | Piperazine replacement ZH | R-Substituent of Product of Ex. 21 | Compound I obtained ($R_1$=H; W and X=F) R | Z |
|---|---|---|---|---|
| 15. | morpholine | o-fluorophenyl | o-fluorophenyl | morpholinyl |
| 16. | morpholine | 2,4-dihydrophenyl | 2,4-dihydrophenyl | morpholinyl |
| 17. | morpholine | p-methoxyphenyl | p-methoxyphenyl | morpholinyl |
| 18. | thiomorpholine | o-fluorophenyl | o-fluorophenyl | thiomorpholinyl |
| 19. | thiomorpholine | 2-hydroxyphenyl | 2-hydroxyphenyl | thiomorpholinyl |
| 20. | 2,6-dimethyl-piperazine | o-fluorophenyl | o-fluorophenyl | 3,5-dimethyl-1-piperazinyl |
| 21. | homopiperazine | o-fluorophenyl | o-fluorophenyl | homopiperazinyl |
| 22. | dimethylamine | p-methylphenyl | p-methylphenyl | dimethylamino |
| 23. | 2,2-dimethyl-hydrazine | o-fluorophenyl | o-fluorophenyl | 2,2-dimethyl-hydrazyl |
| 24. | thiomorpholine | 2-hydroxy-4-fluorophenyl | 2-hydroxy-4-fluorophenyl | thiomorpholinyl |
| 25. | morpholine | p-cyanophenyl | p-cyanophenyl | morpholinyl |
| 26. | morpholine | p-aminophenyl | p-aminophenyl | morpholinyl |
| 27. | piperidine | p-methyl-mercaptophenyl | p-methyl-mercaptophenyl | piperidinyl |
| 28. | piperazine | 3-chloro-4-hydroxyphenyl | 3-chloro-4-hydroxyphenyl | piperazinyl |
| 29. | N-methylpiperazine | 3-chloro-4 hydroxyphenyl | 3-chloro-4-hydroxyphenyl | 4-methylpiperazinyl |
| 30. | N-acetylpiperazine | p-fluorophenyl | p-fluorophenyl | 4-acetylpiperazinyl |
| 31 | N-propionyl-piperazine | p-fluorophenyl | p-fluorophenyl | 4-propionyl-piperazinyl |
| 32. | piperazine | 3,4-methylene-dioxyphenyl | 3,4-methylene-dioxyphenyl | piperazinyl |
| 33. | N-methyl-piperazine | 3,4-methylene-dioxyphenyl | 3,4-methylene-dioxyphenyl | 4-methyl-piperazinyl |

Example 23

In the described fashion of Example 5(b), the compounds 11 and 12 in Example 22 can be converted to:

(a) 1-o-fluorophenyl-6,8-difluoro-1,4-dihydro-4-oxo-7-(3-amino-1-pyrrolidinyl)-quinoline-3-carboxylic acid.

(b) 1-p-methylphenyl-6,8-difluoro-1,4-dihydro-4-oxo-7-3-amino-1-pyrrolidinyl)-quinoline-3-carboxylic acid.

**Claims**

1. A compound having the formula:

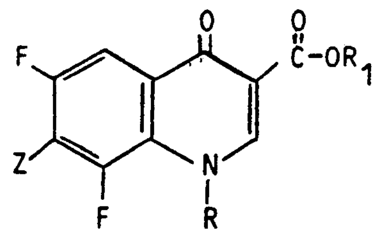

wherein $R_1$ is hydrogen or a carboxy protecting group; R is phenyl having the formula:

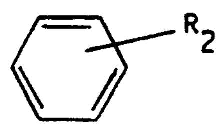

wherein $R_2$ is methylenedioxy or one or two substituents independently selected from hydrogen, halogen, nitro, carboxyl, cyano, $C_1$ to $C_6$ alkyl, hydroxy-substituted $C_1$ to $C_6$ alkyl, halo substituted $C_1$ to $C_6$ alkyl, a substituent having the formula:

$$—Y—R_3$$

wherein —Y— is —O— or —S— and $R_3$ is hydrogen or $C_1$ to $C_6$ alkyl, and a substituent of the formula:

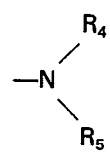

wherein $R_4$ and $R_5$ are independently hydrogen or $C_1$ to $C_6$ alkyl; and, Z is selected from 1) an aliphatic heterocyclic ring having the structure:

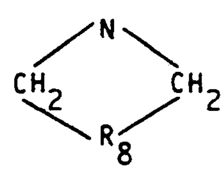

wherein $R_8$ is $—CH_2—CH_2—$ or $—CH_2—R_9—CH_2—$ wherein $R_9$ is selected from the group consisting of —S, —O—, —NH—, $—CH_2—$ and $—NH—CH_2—$; 2) substituted derivatives of said aliphatic heterocyclic ring having one or two substituents independently selected from $C_1$ to $C_6$ alkyl, $C_1$ to $C_6$ hydroxyalkyl, hydroxy, alkanoyl having 1 to 6 carbon atoms, alkanoylamino having 1 to 6 carbon atoms and a substituent of the formula:

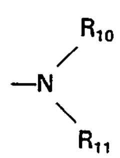

wherein $R_{10}$ and $R_{11}$ are each independently selected from hydrogen and $C_1$ to $C_6$ alkyl; and 3) a substituent of the formula:

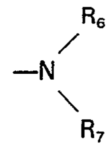

wherein $R_6$ is hydrogen, $C_1$ to $C_{10}$ alkyl or hydroxy-substituted $C_1$ to $C_{10}$ alkyl, and $R_7$ is selected from $C_1$ to $C_{10}$ alkyl, hydroxy-substituted $C_1$ to $C_{10}$ alkyl, $—NH_2$, a mono-($C_1$ to $C_6$) alkylamino and a di-($C_1$ to $C_6$) alkylamino; and pharmaceutically acceptable salts thereof.

2. A compound as defined in claim 1 wherein the aliphatic heterocyclic ring is selected from the group consisting of piperazinyl, piperidinyl, pyrrolidinyl, morpholino, thiomorpholino and homopiperazinyl.

3. A compound as defined in Claim 1 wherein Z is piperazinyl, $C_1$ to $C_6$ alkanoyl piperazinyl or $C_1$ to $C_6$ alkyl piperazinyl.

4. A compound as defined in Claim 1 wherein R is phenyl or substituted phenyl wherein the substituent on the phenyl group is methylenedioxy or one or two substituents independently selected from $C_1$ to $C_6$ alkyl, halo and hydroxy.

5. A compound as defined in claim 1 wherein R is o,p difluorophenyl.

6. A compond as defined in claim 3 wherein Z is 4-methyl-1-piperazinyl.

7. A compound as defined in claim 1 wherein R is p-fluorophenyl.

8. A compound as defined in claim 1 wherein Z is 3-aminopyrrolidin-1-yl.

9. A compound as defined in claim 1, 3 or 4 wherein R is p-fluorophenyl, Z is piperazinyl and $R_1$ is hydrogen.

10. A compound as defined in claim 1, 3 or 4 wherein R is p-fluorophenyl, Z is 4-methylpiperazinyl and $R_1$ is hydrogen.

11. A compound as defined in claim 1, 3 or 4 wherein R is p-hydroxyphenyl, Z is piperazinyl and $R_1$ is hydrogen.

12. A compound as defined in claim 1, 3 or 4 wherein R is p-fluorophenyl, Z is 4-acetylpiperazinyl and $R_1$ is hydrogen.

13. A compound as defined in claim 1, 3 or 4 wherein R is phenyl, Z is piperazinyl and $R_1$ is hydrogen.

14. A compound as defined in claim 1, 3 or 4 wherein R is phenyl, Z is 4-methylpiperazinyl and $R_1$ is hydrogen.

15. A compound as defined in claim 1 wherein $R_1$ is hydrogen.

16. A compound as defined in claim 1 wherein Z is an amino group having the formula:

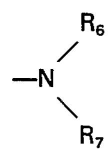

wherein $R_6$ is hydrogen, $C_1—C_{10}$ alkyl or hydroxy-substituted $C_1—C_{10}$ alkyl and $R_7$ is $C_1—C_{10}$ alkyl, hydroxy-substituted $C_1—C_{10}$ alkyl, amino, mono-($C_1—C_4$)alkylamino or di($C_1—C_4$)alkylamino.

17. A composition having antibacterial activity in pharmaceutical dosage form containing a diluent and a compound as defined in claim 1.

18. A composition having antibacterial activity in pharmaceutical dosage form containing a diluent and a compound as defined in claim 3 or 4.

19. A compound as defined in claim 1 for use in treating a bacterial infection in a patient.

20. A compound as defined in claim 3 or 4 for use in treating a bacterial infection in a patient.

**Patentansprüche**

1. Verbindung der Formel

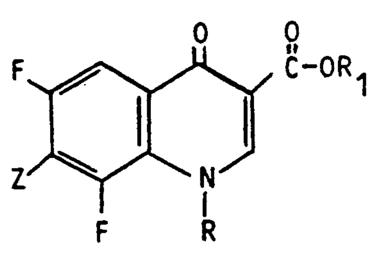

worin $R_1$ Wasserstoff oder eine Carboxyschutzgruppe ist; R Phenyl der Formel ist

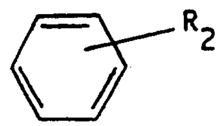

worin $R_2$ Methylendioxy oder ein oder zwei Substituenten ist, die unabhängig aus Wasserstoff, Halogen, Nitro, Carboxyl, Cyano, $C_1$- bis $C_6$-Alkyl, hydroxysubstituiertem $C_1$- bis $C_6$-Alkyl, halogensubstituiertem $C_1$- bis $C_6$-Alkyl, einem Substituenten der Formel

$$—Y—R_3$$

worin —Y— —O— oder —S— ist und $R_3$ Wasserstoff oder $C_1$- bis $C_6$-Alkyl ist, und ein Substituent der Formel

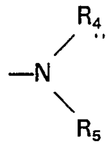

worin $R_4$ und $R_5$ unabhängig Wasserstoff oder $C_1$- bis $C_6$-Alkyl sind, ist; und Z aus 1) einem aliphatischen, heterocyclischen Ring der Struktur

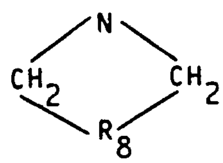

worin $R_8$ $—CH_2—CH_2—$ oder $—CH_2—R_9—CH_2—$ ist, worin $R_9$ aus der aus —S—, —O—, —NH—, $—CH_2—$ und $—NH—CH_2—$ bestehenden Gruppe ausgewählt ist; 2) substituierten Derivaten des aliphatischen heterocyclischen Rings mit ein oder zwei Substituenten, die unabhängig aus $C_1$- bis $C_6$-Alkyl, $C_1$- bis $C_6$-Hydroxyalkyl, Hydroxy, Alkanoyl mit 1 bis 6 Kohlenstoffatomen, Alkanoylamino mit 1 bis 6 Kohlenstoffatomen und einem Substituenten der Formel

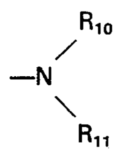

worin $R_{10}$ und $R_{11}$ jeweils unabhängig aus Wasserstoff und $C_1$- bis $C_6$-Alkyl ausgewählt sind; und 3) einem Substituenten der Formel

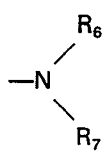

worin $R_6$ Wasserstoff, $C_1$- bis $C_6$-Alkyl oder hydroxysubstituiertes $C_1$- $C_{10}$-Alkyl ist und $R_7$ ausgewählt ist aus $C_1$- bis $C_{10}$-Alkyl, hydroxysubstituierten $C_1$- bis $C_{10}$-Alkyl, $—NH_2$, einem Mono-($C_1$- bis $C_6$)-Alkylamino und einem Di-($C_1$- bis $C_6$)-Alkylamino, ausgewählt ist; sowie pharmazeutisch annehmbare Salze davon.

2. Verbindung, wie in Anspruch 1 definiert, worin der aliphatische heterocyclische Ring aus der aus Piperazinyl, Piperidinyl, Pyrrolidinyl, Morpholino, Thiomorpholino und Homopiperazinyl bestehenden Gruppe ausgewählt ist.

3. Verbindung, wie in Anspruch 1 definiert, worin Z Piperazinyl, $C_1$- bis $C_6$-Alkanoylpiperazinyl oder $C_1$- bis $C_6$-Alkylpiperazinyl ist.

4. Verbindung, wie in Anspruch 1 definiert, worin R Phenyl oder substituiertes Phenyl ist, worin der Substituent an der Phenylgruppe Methylendioxy oder ein oder zwei Substituenten, unabhängig aus $C_1$- bis $C_6$-Alkyl, Halogen und Hydroxy ausgewählt ist.

5. Verbindung, wie in Anspruch 1 definiert, worin R o,p-Difluorphenyl ist.

6. Verbindung, wie in Anspruch 3 definiert, worin Z 4-Methyl-1-piperazinyl ist.

7. Verbindung, wie in Anspruch 1 definiert, worin R p-Fluorphenyl ist.

8. Verbindung, wie in Anspruch 1 definiert, worin Z 3-Aminopyrrolidin-1-yl ist.

9. Verbindung, wie in Anspruch 1, 3 oder 4 definiert, worin R p-Fluorphenyl ist, Z Piperazinyl ist und $R_1$ Wasserstoff ist.

10. Verbindung, wie in Anspruch 1, 3 oder 4 definiert, worin R p-Fluorphenyl ist, Z 4-Methylpiperazinyl ist und $R_1$ Wasserstoff ist.

11. Verbindung, wie in Anspruch 1, 3 oder 4 definiert, worin R p-Hydroxyphenyl ist, Z Piperazinyl ist und $R_1$ Wasserstoff ist.

12. Verbindung, wie in Anspruch 1, 3 oder 4 definiert, worin R p-Fluorphenyl ist, Z 4-Acetylpiperazinyl ist und $R_1$ Wasserstoff ist.

13. Verbindung, wie in Anspruch 1, 3 oder 4 definiert, worin R Phenyl ist, Z Piperazinyl ist und $R_1$ Wasserstoff ist.

14. Verbindung, wie in Anspruch 1, 3 oder 4 definiert, worin R Phenyl ist, Z 4-Methylpiperazinyl ist und $R_1$ Wasserstoff ist.

15. Verbindung, wie in Anspruch 1 definiert, worin $R_1$ Wasserstoff ist.

16. Verbindung, wie in Anspruch 1 definiert, worin Z eine Aminogruppe der Formel ist

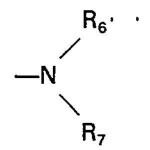

worin $R_6$ Wasserstoff, $C_1$—$C_{10}$-Alkyl oder hydroxysubstituiertes $C_1$—$C_{10}$-Alkyl ist und $R_7$ $C_1$—$C_{10}$-Alkyl, hydroxysubstituiertes $C_1$—$C_{10}$-Alkyl, Amino, Mono-($C_1$—$C_4$)-alkylamino oder Di-($C_1$—$C_4$)-Alkylamino ist.

17. Zusammensetzung mit antibakterieller Wirkung in pharmazeutischer Dosierungsform, welche ein Verdünnungsmittel und eine Verbindung, wie in Anspruch 1 definiert, enthält.

18. Zusammensetzung mit antibakterieller Wirkung in pharmazeutischer Dosierungsform, welche ein Verdünnungsmittel und eine Verbindung, wie in Anspruch 3 oder 4 definiert, enthält.

19. Verbindung, wie in Anspruch 1 definiert, zur Verwendung bei der Behandlung einer bakteriellen Infektion in einem Patienten.

20. Verbindung, wie in Anspruch 3 oder 4 definiert, zur Verwendung bei der Behandlung einer bakteriellen Infektion in einem Patienten.

**Revendications**

1. Un composé ayant la formule:

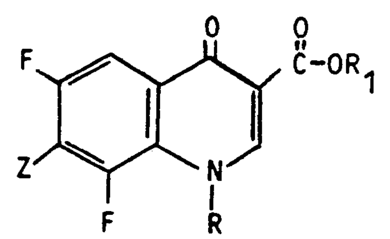

dans laquelle $R_1$ est un atome d'hydrogène ou un groupe protecteur du groupe carboxyle; R est un groupe phényle ayant la formule:

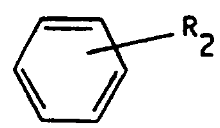

dans laquelle $R_2$ est un groupe méthylènedioxy ou un ou deux substituants choisis indépendamment parmi les suivants: hydrogène, halogène, nitro, carboxyle, cyano, $C_1$—$C_6$ alkyle, hydroxy-$C_1$—$C_6$ alkyle, halo-$C_1$—$C_6$ alkyle, un substituant ayant la formule:

$$—Y—R_3$$

dans laquelle Y est —O— ou —S— et $R_3$ est l'hydrogène ou $C_1$—$C_6$ alkyle, et un substituant de formule:

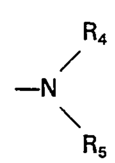

dans laquelle $R_4$ et $R_5$ désignent indépendamment l'hydrogène ou $C_1$—$C_6$ alkyle; et Z est choisi parmi les suivants: 1) un noyau hétérocyclique aliphatique ayant la structure:

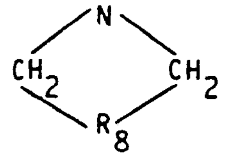

dans laquelle $R_8$ est $—CH_2—CH_2—$ ou $—CH_2—R_9—CH_2—$ dans laquelle $R_9$ est choisi dans le groupe constitué par —S, —O—, —NH—, $—CH_2—$ et $—NH—CH_2—$; 2) les dérivés substitués dudit noyau hétérocyclique aliphatique ayant un ou deux substituants choisis indépendamment dans le groupe suivant: $C_1—C_6$ alkyle, $C_1—C_6$ hydroxyalkyle, hydroxy, alcanoyle de 1 à 6 atomes de carbone, alcanoylamino de 1 à 6 atomes de carbone et un substituant de formule:

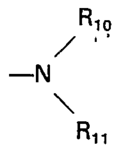

dans laquelle $R_{10}$ et $R_{11}$ sont choisis chacun indépendamment parmi l'hydrogène et $C_1—C_6$ alkyle; et 3) un substituant de formule:

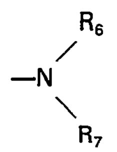

dans laquelle $R_6$ est l'hydrogène, $C_1—C_{10}$ alkyle ou $C_1—C_{10}$ alkyle à substitution hydroxy et $R_7$ est choisi parmi les suivants: $C_1—C_{10}$ alkyle, $C_1—C_{10}$ alkyle à substitution hydroxy, $—NH_2$, mono-($C_1—C_6$) alkylamino et di-($C_1—C_6$) alkylamino; et les sels pharmaceutiquement acceptables dudit composé.

2. Un composé selon la revendication 1, selon lequel le noyau hétérocyclique aliphatique est choisi dans le groupe comprenant les suivants: pipérazinyle, pipéridinyle, pyrrolidinyle, morpholino, thiomorpholino et homopipérazinyle.

3. Un composé selon la revendication 1, selon lequel Z est un groupe pipérazinyle, $C_1—C_6$ alcanoylpipérazinyle ou $C_1—C_6$ alkylpipérazinyle.

4. Un composé selon la revendication 1, selon lequel R est un groupe phényle ou un groupe phényle substitué dans lequel le substituant sur le groupe phényle est un groupe méthylènedioxy ou un ou deux substituants choisis indépendamment parmi $C_1—C_6$ alkyle, halo et hydroxy.

5. Un composé selon la revendication 1, selon lequel R est le groupe o,p-difluorophényle.

6. Un composé selon la revendication 3, selon lequel Z est le groupe 4-méthyl-1-pipérazinyle.

7. Un composé selon la revendication 1, selon lequel R est le groupe p-fluorophényle.

8. Un composé selon la revendication 1, selon lequel Z est le groupe 3-aminopyrrolidine-1-yle.

9. Un composé selon la revendication 1, 3 ou 4, selon lequel R est le groupe p-fluorophényle, Z est le groupe pipérazinyle et $R_1$ est l'hydrogène.

10. Un composé selon la revendication 1, 3 ou 4, selon lequel R est le groupe p-fluorophényle, Z est le groupe 4-méthylpipérazinyle et $R_1$ est l'hydrogène.

11. Un composé selon la revendication 1, 3 ou 4, selon lequel R est le groupe p-hydroxyphényle, Z est le groupe pipérazinyle et $R_1$ est l'hydrogène.

12. Un composé selon la revendication 1, 3 ou 4, selon lequel R est le groupe p-fluorophényle, Z est le groupe 4-acétylpipérazinyle et $R_1$ est l'hydrogène.

13. Un composé selon la revendication 1, 3 ou 4, selon lequel R est le groupe phényle, Z est le groupe pipérazinyle et $R_1$ est l'hydrogène.

14. Un composé selon la revendication 1, 3 ou 4, selon lequel R est le groupe phényle, Z est le groupe 4-méthylpipérazinyle et $R_1$ est l'hydrogène.

15. Un composé selon la revendication 1, selon lequel $R_1$ est l'hydrogène.

16. Un composé selon la revendication 1, selon lequel Z est un groupe amino ayant la formule:

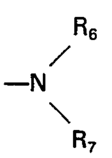

dans laquelle $R_6$ est un atome d'hydrogène, un groupe $C_1—C_{10}$ alkyle ou hydroxy-$C_1—C_{10}$ alkyle et $R_7$ est un groupe $C_1—C_{10}$ alkyle, hydroxy-$C_1—C_{10}$ alkyle, amino, mono-($C_1—C_4$) alkylamino ou di-($C_1—C_4$) alkylamino.

17. Une composition ayant une activité antibactérienne sous forme de dosage pharmaceutique contenant un diluant et un composé tel que défini dans la revendication 1.

18. Une composition ayant une activité antibacterienne sous forme de dosage pharmaceutique contenant un diluant et un composé tel que défini dans la revendication 3 ou 4.

19. Un composé tel que défini dans la revendication 1 à utiliser dans le traitement d'une infection bactérienne chez un patient.

20. Un composé tel que défini dans la revendication 3 ou 4 à utiliser dans le traitement d'une infection bactérienne chez un patient.